(19) Europäisches Patentamt
European Patent Office
Office européen des brevets

(11) Numéro de publication: **0 317 544**
**A1**

(12) # DEMANDE DE BREVET EUROPEEN

(21) Numéro de dépôt: **88870172.9**

(22) Date de dépôt: **16.11.88**

(51) Int. Cl.[4]: **C 12 N 9/96**
C 12 N 11/06
// C12N9/04

(30) Priorité: **16.11.87 BE 8701294**

(43) Date de publication de la demande:
**24.05.89 Bulletin 89/21**

(84) Etats contractants désignés:
**AT BE CH DE ES FR GB GR IT LI LU NL SE**

(71) Demandeur: **Remacle, Jose**
**14 Ch. des Pierres**
**B-5730 Malonne (BE)**

(72) Inventeur: **Remacle, Jose**
**14 Ch. des Pierres**
**B-5730 Malonne (BE)**

(54) **Méthode de modification du micro-environnement de l'enzyme par greffage de chaîne peptidiques. Utilisation pour la réalization de bioréacteurs enzymatiques nécessitant la régénération de cofacteur.**

(57) L'invention concerne une méthode de modification du micro-environnement de l'enzyme par greffage de chaînes peptidiques. La longueur, la composition et les propritétés de ces chaînes peptidiques peuvent interagir avec la surface de l'enzyme et peuvent aussi être activées à leur extrémité de manière à les fixer sur un support insoluble ou de les faire réagir dans une réaction de copolymérisation pour former un gel dans lequel l'enzyme sera immobilisée dans un micro-environnement déterminé. Un exemple montrant les modifications de l'alcool déshydrogénase, permettant la régénération du NADH pour la synthèse d'alanine dans un bioréacteur enzymatique, illustre les possibilités de l'invention.

EP 0 317 544 A1

## Description

**Méthode de modification du micro-environnement de l'enzyme par greffage de chaînes peptidiques. Utilisation pour la réalisation de bioréacteurs enzymatiques nécessitant la régénération de cofacteur.**

## 2.- DOMAINE DE L'INVENTION

L'invention concerne un procédé qui permet la modification du micro-environnement de l'enzyme, ce qui entraîne des modifications dans les propriétés de l'enzyme, certaines de celles-ci étant très avantageuses pour l'utilisation des enzymes dans les usages industriels comme par exemple : changement du pH optimum d'activité, stabilité accrue à certains pH, stabilité thermique plus grade, résistance plus importante aux inactivations chimiques etc... Cette invention permet une meilleure utilisation pratique de ces enzymes en solution mais aussi après immobilisation. Ce procédé peut être appliqué pour les systèmes enzymatiques nécessitant la régénération de cofacteurs utilisés pour des synthèses de produits chimiques, pharmaceutiques ou agro-alimentaires.

## 3.- ETAT DE LA TECHNIQUE

Contrairement à la plupart des catalyseurs chimiques, les enzymes sont des catalyseurs extrêmement puissants et spécifiques. Ces deux propriétés leur confèrent un important potentiel d'utilisation, tant dans les domaines analytiques (comme électrodes enzymatiques) qu'industriels (Bioréacteurs enzymatiques) voire thérapeutiques (Monsan, les enzymes - Production et utilisations industrielles, Gauthier-Villars, 1982).

Cependant, les enzymes du fait de la relation très stricte entre leur structure tridimentionnelle et leur activité, et de la présence de groupement pouvant réagir chimiquement, sont relativement instables. Une série de procédés et d'inventions ont donc été décrits afin de les stabiliser. On peut par exemple ajouter à l'enzyme des additifs chimiques qui stabilisent plus ou moins bien l'enzyme en solution E.P.A.O.-072-581; E.P.A.-0-074-237; E.D.B.-080-304). On peut aussi conjuguer l'enzyme à un polymère en présence d'hydrolysats d'enzymes qui ont une action protectrice (EP-A-2-022-145). On peut diminuer la mobilité des enzymes par la présence de gélatine (EP-A-2-344-568) ou fixer l'enzyme de manière covalente à un polymère ayant des groupes latéraux pouvant se lier de manière covalente sur des groupements latéraux de l'enzyme (EP-B-0-049-475).

A côté de ces travaux réalisés sur les enzymes en solution, on a essayé de modifier les supports permettant d'insolubiliser les enzymes afin de les stabiliser : par exemple en utilisant un mélange de polyamine à longue chaîne, de Tannin et d'un agent de couplage (EP-A-0-053-764) ou par entrappement de l'enzyme dans la matrice d'un gel de polysaccharride ayant des groupements aminés (EP-A-0-056-111).

Parallèlement à ces inventions très précises se sont développées des recherches plus théoriques mettant en lumière l'importance des interactions de l'enzyme avec son micro-environnement. Une revue à ce sujet a été réalisée en 1971 par Katchalski et al. (Adv. Enzymol., 33, 445-536). Depuis lors, plusieurs travaux ont montré le caractère favorable d'un environnement hydrophile sur la stabilisation d'une enzyme en solution; cette propriété pouvant être obtenue par la présence de polymères hydrophiles (Theorell et Tatemoto, 1971, Arch. Biochem. Biophys., 143, 354-358; Dikov et coll., Biokhimiya, 1984, 49 (9), 1431-1440; Henley et Sadama, Ann. N.Y. Acad. Sci., 1984, 434, 64-69; Bioengin., 1984, 26, 959-969; Arakawa et Timasheff,Biophys. J., 1985, 47, 411-414) ou de chaînes polysaccharidiques (Lenders et Crichton, Biotechnol, Bioengin., 1984, 26, 1343-1351). De manière plus fondamentale, l'intérêt de rendre l'extérieur de la protéine plus hydrophile a été étudié de manière théorique par Bigelow (J. Theor. Biol., 1967, 187-211) et par Dill (Biochemistry, 1985, 24, 1501-1509) dans le cadre de l'étude des forces de stabilisation de la structure tertiaire des protéines. Cette approche a été confirmée par les études de Stellwagen (Ann. N.Y. Acad. Sci., 1984, 434, 1-6) dans le cadre d'une stratégie systématique pour la mutagenèse dirigée.

Ainsi donc le contrôle précis du micro-environnement des enzymes est d'un enjeu capital pour permettre le développement des enzymes comme outils de bioréacteurs ou de biosenseurs car il permettre de moduler les propriétés des enzymes et notamment leur stabilité. Ce dernier point est important pour une multitude d'applications et notamment pour l'utilisation d'enzymes particulièrement instables qui utilisent les cofacteurs.

L'invention telle qu'elle est décrite ci-dessus est un procédé qui permet de moduler l'environnement de l'enzyme de manière à lui faire acquérir des propriétés particulières en solution mais qui peut aussi servir de base à l'immobilisation de l'enzyme par une méthode respectant au maximum la configuration active de l'enzyme à savoir la liaison par points multiples et la co-polymérisation dans un gel. L'utiliation d'autres supports insolubles est également permise. On peut ainsi utiliser les enzymes sous une forme immobilisée tout en ayant modifié leur microenvironnement.

## 4.- DESCRIPTION DE L'INVENTION

L'idée de base de l'invention sur laquelle se greffent plusieurs développements est de fixer sur l'enzyme une série de bras peptidiques (ou acides aminés) qui modifient le micro-environnement de l'enzyme et lui confèrent de ce fait des propriétés particulières. La justification de l'utilisation de bras de nature peptidique est multiple : d'une part, il existe une très grande variété d'acides aminés naturels ou synthétiques qui ont des propriétés très différentes : caractère hydrophile-hydrophobe, charge positive ou négative, présence de groupement SH etc... Ces acides aminés peuvent être utilisés pour réaliser des chaînes peptidiques de longueur variable. Ces chaînes peptidiques peuvent elles-mêmes interragir avec l'enzyme suivant des interactions non covalentes que l'on rencontre dans les protéines naturelles; ces bras ont également une certaine flexibilité qui est nécessaire à l'enzyme pour son activité et enfin ces bras peuvent être activés à leur extrémité de manière à les fixer sur un support insoluble ou à les faire réagir dans une réaction de copolymorisation pour former un gel dans lequel l'enzyme sera immobilisé. Dans ce dernier cas, on atteint un double but, d'une part la modification du micro-environnement de l'enzyme et donc de ses propriétés et d'autre part son insolubilisation permettant son emploi dans les biosenseurs et bioréacteurs enzymatiques.

Examinons d'abord les propriétés des acides aminés. Il existe 20 acices aminés naturels de base qui sont optiquement actifs et qui sont de la série L. A côté de ces acides aminés naturels, se trouvent les acides aminés synthétiques qui comprennent aussi les isomères optiques de la série D des acides aminés naturels. Ils sont disponibles sur le marché et vendus par la plupart des firmes de produits chimiques (Sigma, Aldrich, Novabiochem). Les groupements aminés et acides carboxyliques étant impliqués dans la liaison peptidique, les propriétés des chaînes peptidiques dépendront essentiellement de la nature des radicaux propres à chaque acide aminé. Un des caractères les plus importants pour expliquer les interactions entre chaînes peptidiques est le caractère plus ou moins hydrophile ou hydrophobe de ce radical. Un tableau comparatif a été établi par Eisenberg 1984 (Ann. Rev. Biochem., 53, 595-623) et ce sont ces données que nous utiliserons dans le travail décrit ici.

Un autre caractère important est la charge portée par ce radical. Celui-ci est facilement connu à partir de la connaissance des différents pK de ces groupements et du pH de la solution.

L'obtention des bras peptidiques peut se réaliser de diverses manières. On peut les synthétiser en une seule réaction à partir des acides aminés libres en solution et obtenir des chaînes peptidiques plus ou moins longues qui peuvent alors être fractionnées suivant leur taille par chromatographie sur tamis moléculaire. Cette méthode est rapide mais elle produit des chaînes peptidiques relativement longues et dont la séquence n'est pas nécessairement connue avec précision puisque l'organisation des acides aminés se fera au hasard sauf si l'on désire obtenir un homopolymère formé d'un même acide aminé.

Les chaînes peptidiques peuvent aussi être obtenues à partir de protéines naturelles par digestion enzymatique. La séparation de ces chaînes peut alors se faire suivant leur taille par chromatographie sur tamis moléculaire, suivant leur charge par séparation sur un gel échangeur d'ions ou suivant leur caractère hydrophile/hydrophobe par chromatographie sur gel hydrophobe. Cette méthode d'obtention des peptides est techniquement facile et permet d'obtenir aussi de grosses quantités de peptides mais si leur propriétés peut être connue, on ne connaît pas nécessairement à l'avance quelle sera leur séquence en acides aminés.

Enfin, il est possible de synthétiser les peptides, acide aminé après acide aminé en utilisant la synthèse en phase solide qui a été introduite par Merrifield (J. Am. Chem. Soc., 1963, 85, 2149-2154; Adv. Enzymol., 1969, 32, 221-296). C'est cette méthode que nous utiliserons dans les exemples ci-après sans exclure les 2 autres procédés. Nous profiterons également de la synthèse par étapes afin d'introduire une homocystéine comme premier acide aminé qui se convertira en thiolactone au moment du traitement de la résine par HF pour libérer le peptide. Cette thiolactone sera alors activée par la pyridine thiol pour générer un pont disulfure qui réagira ultérieurement avec les SH fixés sur l'enzyme préalablement activé.

Dans certains cas, nous avons simplifié la procédure en ajoutant la cystéine comme premier acide aminé de la chaîne et en utilisant son groupement SH pour se fixer sur les points disulfures introduits sur l'enzyme par activation au SPDP (ester succinimique de l'acide 3-(2-pyridyldithio-propionique).

Il existe encore bien d'autres manières d'activer les peptides ou l'enzyme de manière à les faire réagir l'un avec l'autre. Citons par exemple l'activation de l'enzyme à la N-ethyl maléimide et sa réaction ultérieure avec un groupement SH du peptide.

Nous avons testé cette invention sur une enzyme que nous avons prise comme modèle, l'alcool déshydrogénase (ADH) sur laquelle nous avons greffé soit un acide aminé soit une chaîne peptidique afin d'examiner les conséquences de la présence de ce peptide sur les propriétés de l'enzyme. Nous avons aussi profité de la présence de ces peptides pour leur ajouter un groupement vinyl qui a servi pour réaliser une copolymérisation avec un gel de polyacrylamide à 12,5 % final. Les conditions exactes de ces expériences sont données dans un exemple ci-après. L'ensemble de la composition de ces préparations est présenté au tableau 1.

Nous avons alors comparé ces diverses préparations en fonction de différents paramètres caractéristiques de l'enzyme.

a.- Au tableau 2, nous avons indiqué les pH optimum d'activité de l'enzyme. On s'aperçoit que la présence de polyvaline augmente le pH optimum alors que la polysérine le diminue. Dans le cas de la forme immobilisée, tous les pH sont inférieurs à celui de la forme libre de l'enzyme mais il faut noter que ce que l'on mesure sont les pH apparents ; en effet dans le cas d'enzymes immobilisés, il faut établir une correction due au phénomène de diffusion des substrats et des produits et de leur transport facilité à l'intérieur des mailles du gel part la présence du tampon.

b.- Influence sur les constantes cinétiques

L'alcool déshydrogénase étant un enzyme suivant une réaction ordonnée à deux substrats, sa cinétique répond à l'équation :

$$v = \frac{Vmax\,[A]\,[B]}{KiaKb + Ka[B] + Kb[A] + [A].[B]}$$

avec [A] = concentration en NAD+

[B] = concentration en éthanol

Ka et Kb sont les constantes de type Michaelis-Menten correspondant à la dissociation du complexe ternaire du $NAD^+$ et de l'éthanol alors que Kia représente la constante de dissociation du complexe binaire ADH-$NAD^+$ (Wratten et Cleland, 1963, Biochemistry 2, 935-941).

Les valeurs des constantes pour les diverses préparations sont donnés au tableau 3. Nous ne pouvons pas ici interpréter chacune de ces valeurs et nous nous limiterons aux variations les plus importantes. En ce qui concerne le Kia, rappelons que plus le Kia est petit, plus l'affinité de l'enzyme pour la fixation de son premier substrat, le $NAD^+$ est grande. Nous remarquons peu de variation pour les préparations solubles excepté pour la polylysine. On peut expliquer cette baisse d'affinité probablement par une stabilisation de la configuration de l'enzyme due à une interaction de type hydrophile entre la polylysine et les acides aminés hydrophiles de la surface de l'enzyme. Il faut aussi tenir compte de la charge positive de la lysine qui pourrait inhiber la diffusion du $NAD^+$. Dans le cas de Ka et Kb, la fixation des peptides sur l'enzyme augmente les constantes, c'est-à-dire diminue l'affinité de fixation de ces substrats sur les complexes quaternaires de l'enzyme. Dans le cas des enzymes incorporés dans le gel, on constate aussi certains changements mais il faut en plus de l'effet du micro-environnement tenir compte des effets de diffusion. C'est ce dernier paramètre qui explique la valeur très faible du Kia observé pour la poly-lysine-résine. En effet la présence de charges positives enfermées dans le gel doit probablement diminuer la dissociation du complexe enzyme-$NAD^+$ du fait de la charge positive du $NAD^+$.

c- Stabilité des préparations

Il s'agit d'un paramètre important du point de vue pratique puisque l'augmentation de la stabilité des préparations enzymatiques permet d'augmenter la période d'utilisation. Nous avons examiné cette stabilité à basse température (4°C) et à haute température (50°C). En effet aux températures basses, la dénaturation des enzymes résulte presque exclusivement de réactions chimiques alors qu'à haute température les attaques chimiques sont toujours présentes mais elles se superposent aux modifications de conformation de l'enzyme due à l'énergie cinétique élevée des molécules.

Les résultats de stabilité sont exprimés en temps de demi-vie, c'est-à-dire le temps nécessaire à la diminution de la moitié de l'activité. Certaines courbes étant nettement biphasiques elles ont été ajustées par 2 exponentielles (2 exp.). Les résultats sont indiqués au tableau 4.

| | |
|---|---|
| ADH-L | $ADH-NH_2$ |
| ADH-CA | $ADH-NH-CO-CH-CH_2$ |
| ADH-SPDP | $ADH-NH-CO-(CH_2)_2-SH$ |
| ADH-HCY | $ADH-NH-CO-(CH_2)_2-S-S-(CH_2)_2-CH(CO-NH_2)-NH-CO-CH=CH_2$ |
| ADH-SER | $ADH-NH-CO-(CH_2)_2-S-S-(CH_2)_2-CH(CO-NH_2)-NH-(CO-CH(CH_2-OH)-NH)_4-CO-CH=CH_2$ |
| ADH-VAL | $ADH-NH-CO-(CH_2)_2-S-S-CH_2-CH(CO-NH_2)-NH-(CO-CH(CH(CH_3)_2)-NH)_4-CO-CH=CH_2$ |
| ADH-LYS | $ADH-NH-CO-(CH_2)_2-S-S-CH_2-CH(CO-NH_2)-NH-(CO-CH((CH_2)_4-NH_2)-NH)_4-CO-CH=CH_2$ |
| ADH-CA-PAA | $ADH-NH-CO-CH_2-CH_2$-RESINE |
| ADH-HCY-PAA | $ADH-NH-CO-(CH_2)_2-S-S-(CH_2)_2-CH(CO-NH_2)-CO-CH_2-CH_2$-RESINE |
| ADH-SER-PAA | $ADH-NH-CO-(CH_2)_2-S-S-(CH_2)_2-CH(CO-NH_2)-NH-(CO-CH(CH_2-OH)-NH)_4-CO-CH_2-CH_2$-RESINE |
| ADH-VAL-PAA | $ADH-NH-CO-(CH_2)_2-S-S-CH_2-CH(CO-NH_2)-NH-(CO-CH(CH(CH_3)_2)-NH)_4-CO-CH_2-CH_2$-RESINE |
| ADH-LYS-PAA | $ADH-NH-CO-(CH_2)_2-S-S-CH_2-CH(CO-NH_2)-NH-(CO-CH((CH_2)_4-NH_2)-NH)_4-CO-CH_2-CH_2$-RESINE |

Tableau 1.- : Liste des diverses modifications apportées à l'enzyme (ADH) par addition de peptides qui ont pu alors servir pour réaliser une copolymérisation avec un gel d'acrylamide (résine).

Tableau 2

| Préparation | Température optimale (°C) forme soluble | forme immobilisée |
|---|---|---|
| ADH-L | 45 | - |
| ADH-CA | 40 | 35 |
| ADH-SPDP | 40 | - |
| ADH-HCY | 40 | 35 |
| ADH-SER | 45 | 35 |
| ADH-VAL | 40 | 35 |
| ADH-LYS | 45 | 30 |

Valeurs de la température optimale d'activité des diverses préparations enzymatiques.

Tableau 3

| Préparation | Température optimale (°C) forme soluble | forme immobilisée |
|---|---|---|
| ADH-L | 45 | - |
| ADH-CA | 40 | 35 |
| ADH-SPDP | 40 | - |
| ADH-HCY | 40 | 35 |
| ADH-SER | 45 | 35 |
| ADH-VAL | 40 | 35 |
| ADH-LYS | 45 | 30 |

Valeurs des constantes cinétiques des diverses préparations d'ADH.

Tableau 4

| Préparations | demi-vie à 4°C (jours) | modèle | demi-vie à 50°C (min) | modéle |
|---|---|---|---|---|
| ADH-L | 5,1 | 1 exp. | 30,2 | 2 exp. |
| ADH-CA | 4,7 | 1 exp. | 47,6 | 2 exp. |
| ADH-SPDP | 18,4 | 1 exp. | 203,4 | 1 exp. |
| ADH-HCY | 3,6 | 1 exp. | 52,7 | 2 exp. |
| ADH-SER | 32,2 | 2 exp. | 61,6 | 1 exp. |
| ADH-VAL | 5,3 | 2 exp. | 15,6 | 1 exp. |
| ADH-LYS | 27,3 | 2 exp. | 20,0 | 1 exp. |
| ADH-CA-PAA | 5,5 | 1 exp. | 107,0 | 1 exp. |
| ADH-HCY-PAA | 4,4 | 1 exp. | 72,4 | 1 exp. |
| ADH-SER-PAA | 1,8 | 1 exp. | 67,3 | 1 exp. |
| ADH-VAL-PAA | 0,9 | 1 exp. | 61,3 | 1 exp. |
| ADH-LYS-PAA | 0,8 | 1 exp. | 4,1 | 1 exp. |

Valeurs des temps de demi-vie des différentes préparations de l'ADH à 4°C et à 50°C calculées à partir d'un modèle à 1 exponentielle (1 exp.) ou à 2 exponentielles (2 exp.).

Si l'on examine d'abord les valeurs obtenues à 4°C, on s'aperçoit que trois préparations stabilisent fortement l'enzyme : l'enzyme activé au SPDP qui contient des groupements SH, l'enzyme polyserine et polylysine. Dans le cas du SPDP, l'explication est simple, la présence de groupements Sulphydryles à la surface de la protéine protège le groupement SH natif de l'enzyme qui est nécessaire à l'activité de l'enzyme. Il

y aurait donc une protection due à une compétition entre les SH supplémentaires et le SH du site actif pour des agents chimiques toxiques ou pour une oxydation. On peut imaginer le même raisonnement pour la lysine et la sérine qui pourraient être attaquées et protéger ainsi les acides aminés propres de l'enzyme. Cependant, on ne peut pas exclure que vu leur caractère hydrophile, ils ne stabilisent l'extérieur de l'enzyme par interaction forte avec le périphérie hydrophile de l'enzyme et ne diminuent de cette manière un changement de configuration consécutive à une attaque chimique éventuelle. On retrouve en effet une même stabilisation à 50°C pour le SPDP-SH et la polysérine qui peut s'expliquer de manière semblable; l'instabilité due à la lysine est plus difficile à expliquer : elle pourrait venir d'une déformation locale de la structure de la protéine due à la chaîne très chargée de la polylysine ou à un rôle de la lysine dans une réaction de catalyse acide-base attaquant des résidus comme l'asparagine ou la glutamine. Si notre raisonnement est correct, nous devrions pouvoir protéger encore davantage les enzymes en leur ajoutant des acides aminés identiques à ceux contenus dans le site actif et qui sont sensibles aux attaques comme l'histidine, on pourrait aussi les associer à d'autres résidus très hydrophiles et contenant aussi des SH.

Les mêmes raisonnements sont valables pour les résultats obtenus pour les préparations immobilisées bien que la stabilité à 4°C soit inférieure à celle obtenue pour les enzymes en solution.

## 5. EXEMPLE D'ACTIVATION DE L'ADH PAR UNE POLYSERINE ET COPOLYMERISATION DANS UN GEL DE POLYACRYLAMIDE.

### A. Synthèse de l'oligo-peptide en phase solide (cfr Fig. 3).

**1.- Couplage du premier acide aminé**

Le principe retenu est celui de la synthèse en phase solide (Atherton et al., 1979), Bioorganic therm., 8, 251-370). La résine est un polystyrène-divinylbenzène activé par des groupements méthyle benzhydrylamine (MBHA). Suite à la procédure d'activation, elle se trouve sous forme de chlorhydrate : il convient de restaurer les groupements aminés libres avant de coupler le premier acide aminé; ceci est réalisé au moyen des rinçages suivants :

2 x 2 vol $CH_2Cl_2$ pour regonfler la résine
2 x 2 vol triéthylamine (NEt3) à 10 % dans $CH_2Cl_2$, pour déprotoner l'amine
1 x 2 vol $CH_2Cl_2$ pour rincer la $NEt_3$
2 x 2 vol Méthanol, pour dégonfler la résine et laver le volume mort
2 x 2 vol $CH_2Cl_2$ pour regonfler la résine.

A ce moment, la résine se trouve sous forme aminée. Comme le degré d'activation est de l'ordre de 0,85 à 1 méq MBHA/g de résine et que les meilleurs résultats sont obtenus pour un maximum de 0,3 méq de peptide/g de résine, le premier acide aminé est greffé en défaut et les sites excédentaires sont inactivés (voir le mode opératoire correspondant ci-dessous : "Acétylation des groupements de la résine non substitués"). Le degré d'activation de la résine est estimé par le test de GISIN (Anal. Clin. Acta, 1972, 58, 248-249).

Dans ce cas-ci, le premier acide aminé a toujours été une homocystéine (HCy). Le soufre est protégé par un groupement tertio butyle (terBn) et l'amine est protégée, classiquement, par un groupe tertio butyloxycarbonyle (Boc). Ainsi pour 6 g de résine, on dissout 1,5 mmoles de Boc HCy-StBu et 1,5 mmoles d'hydroxybenzotriazole (HOBt), un activateur dans quelques ml de chlorure de méthylène et 4 ml de diméthyl formamide (DMF, distillé) pour avoir une dissolution complète de l'HOBt. Immédiatement avant le couplage, on ajoute à cette solution 1,5 ml de dicyclohexyl carbodiimide (DCC) 2 M dans du chlorure de méthylène et on filtre le tout sur coton directement dans le réacteur contenant la résine. On complète avec du chlorure de méthylène, de manière à avoir une suspension bien liquide et on incube sous agitation (mise en rotation du réacteur à 15-20 rpm) pendant environ 3-4 h (Erickson & Merrifield, 1976, The Protein, 2, 255-527).

**2.- Acétylation des groupements de la résine non substitués**

Pour saturer la résine, on acétyle les groupements MBHA en excès. A la fin du premier couplage, on lave la résine successivement par :

1 x 2 vol $CH_2Cl_2$
2 x 2 vol Méthanol
2 x 2 vol $CH_2Cl_2$

Ensuite, on acétyle au moyen de 20 ml $CH_2CL_2$ + 4 ml d'anhydride acétique + 80 µl de N-methyl imidazole (FLUKA, Suisse) agissant comme catalyseur. On incube 1 h sous agitation, avant de laver de la même manière que ci-dessus. On vérifie que la protection est complète au moyen du test de Kaiser et al. (Anal. Biochem., 34, 595-598, 1970), qui doit être négatif, sinon la réaction est répétée.

**3.- Greffage des acides aminés suivants**

La suite des opérations se répète autant de fois qu'il le faut pour synthétiser l'ensemble du peptide. A la fin du premier couplage, on rince comme indiqué ci-dessus, et on vérifie au moyen du test de Kaiser que la réaction est complète. Ensuite, on réalise le déblocage de l'amine terminale (protégée par un groupement Boc) au moyen de 2 vol d'acide trifluoracétique (TFA) à 50 % dans le $CH_2Cl_2$, pendant 20 min, sous agitation

modérée. Ensuite, on lave de la manière suivante :
3 x 2 vol $CH_2Cl_2$
1 x 2 vol Triéthylamine ($NET_3$) à 10 % dans $CH_2Cl_2$
1 x 2 vol $CH_2Cl_2$
1 x 2 vol NET″ à 10 % dans $CH_2Cl_2$
1 x 2 vol $CH_2Cl_2$
2 x 2 vol Méthanol
2 x 2 vol $CH_2Cl_2$

A ce moment, le test de Kaiser doit être positif. Le test de Gisin permet alors de déterminer la quantité d'acides aminés couplés sur la résine. On procède ensuite de la même manière pour coupler les acides aminés suivants, si ce n'est qu'on peut utiliser un excès quantitatif (2 à 3 fois par rapport au nombre de sites de couplage).

**4.- Déprotection du peptide et clivage de la résine**

Après le dernier déblocage, la résine est lavée au méthanol et séchée sous vide. On l'introduit ensuite dans un réacteur en Teflon, ainsi que 1,5 ml d'anisole et 0,25 ml de dimethylsulfure par g de résine, afin de piéger les carbocations formés au cours de la réaction. Le réacteur est refroidi dans un bain d'azote liquide, et de l'acide fluorhydrique (HF) y est condensé sous vide, à raison de 10 ml par g de résine. Le réacteur est maintenu sous agitation pendant 40 min à -20°C et encore 40 min à 0°C. Ensuite on chasse le HF.

Cette opération permet d'atteindre 3 objectifs simultanément. D'une part, on clive le peptide de la résine (Doscher, Meth. Enzymol., 1977, 47, 578-617) d'autre part, on déprotège toutes les chaînes latérales (Bodanszky, The practice of peptide synthesis, Springer-Verlag, Berlin, 1984) et enfin, l'Hcy terminale est cyclisée en thiolactone (Chassaing et coll., Tetrahedron Lett., 1986, 26, 623-626),

La résine avec les peptides adsorbés est rincée à l'éther, ensuite le peptide est extrait au moyen d'acide acétique 10 % dégazé. Cette solution est lyophylisée.

La purification ultérieure du peptide a été réalisée par chromatographie de partition sur Sephadex G 25 dans un solvant biphasique butanol/acide acétique/eau : 4/1/5, dans une colonne de 1 m x 2 cm (Yamashiro, nature, 1964, 201 (4914), 76-77). Les pics d'élution sont mesurés à 254 nm. La purification est suivie à chaque étape par chromatographie sur couche mince et HPLC.

**B- Activation du peptide (amino-terminale) au chlorure d'acryloyle**

Après purification des différents peptides synthétisés, on procède à l'activation amino-terminale du peptide au moyen de chlorure d'acryloyle.

Pour ce faire, on incube dans 6 ml de chlorure de méthylène et 2,5 ml de méthanol 4 mmoles de peptide avec 4 mmoles de chlorure d'acryloyle et 8 mmoles de $NET_3$, sous agitation, pendant 1 h à température ambiante.

On peut vérifier le degré d'avancement de la réaction par chromatographie sur couche mince (CCM) sur plaque analytique activée (F254, Merck), dans le système de solvants suivant : chloroforme : 95/méthanol : 5/ ; éthanol : 0,2. En général, le produit est contaminé par un peu de peptide de départ qui n'a pas réagi. On réalise donc une purification par chromatographie sur colonne de silice (Kieselgel 60, Merck) de 18 x 4 cm, sous un flux du même système de solvants que pour la CCM analytique. La pureté du pic obtenu est vérifiée par CCM.

Le solvant d'élution est alors éliminé à l'évaporateur rotatif et le peptide est repris dans un volume minimum d'acide acétique 10 % avant d'être congelé dans un bain d'azote liquide et lyophilisé.

**C. Activation du soufre carboxy-terminal**

La thiolactone formée au cours du clivage du peptide de la résine permet de protéger le soufre de l'homocystéine terminale au cours de l'activation par le chlorure d'acryloyle. Il convient dès lors de rouvrir la thiolactone afin de régénérer un soufre libre. On active donc la thiolactone par un thiol activé (pyridine thiol) de manière à générer ici un pont disulfure susceptible d'être réduit ultérieurement par un thiol de la protéine pour former la liaison peptide-protéine (Lavielle et coll., Peptides-Proceedings of the 9th American Peptide Symposium, Deber, C.M., Hruby, V.J., Kopple K.D., eds), Price Chemical Company, Rockoford, USA, 1985, 35-38).

Pour réaliser cette expérience, on introduit dans un bicol de 30 ml le peptide semi-activé purifié ainsi qu'un excès molaire de 1,25 de 2-mercaptopyridine et de 5 fois de ferricyanure de potassium. Ces produits sont séchés complètement sous vide.

La réaction se réalise dans environ 20 ml d'ammoniac anhydre liquide obtenu par double distillation sur sodium et condensation dans le réacteur (Chassaing et coll., 1986). Après 1 h de réaction à -55°C (bain de DMF et d'azote liquide), on chasse l'ammoniac complètement et sèche les produits sous vide.

On procède ensuite à une extraction au chlorure de méthylène, avec éventuellement un peu de méthanol pour éliminer l'excès de pyridine thiol et sa forme oxydée avant d'extraire le peptide et de filtrer sur tamis moléculaire Sephadex G25 (colonne de 2 cm x 1 m) sous une solution d'acide acétique à 10 %. Le contenu du pic d'élution correspondant est lyophilisé.

Une purification éventuellement nécessaire se fait sur colonne de silice dans le système de solvants déterminé par chromatographie sur couche mince. Ensuite, le pic est récupéré, les solvants sont évaporés et le produit est lyophilisé dans de l'acide acétique à 10 %.

La structure du produit ainsi obtenu est alors confirmée par ses caractéristiques de migration et de marquage en chromatographie sur couche mince et par son signal en spectrométrie infra-rouge et RMN (250 MHz).

**D. Activation de l'enzyme au SPDP**

Le mode opératoire est le suivant : on prépare une solution 20 mM SPDP dans l'éthanol absolu. L'ADH (6 mg pour 0,6 ml) est dissous dans un tampon 0,1 M $Na_2HPO_4$ pH 7,5 contenant 0,1 M NaCl. Après dosage de l'activité, on active l'enzyme en ajoutant 4 x 10 µl de la solution de SPDP et en incubant 30 min à température ambiante, sous agitation modérée. On sépare ensuite les produits de la réaction par filtration sur une colonne de gel Sephadex G 25 (Pharmacia Fine Chemicals, Suède) de 17 x 0,8 cm. On élue au moyen de tampon 0,1 M TRIS-HCl, 0,1 M NaCl, pH 6,5.

Après chromatographie, on obtient trois pics, dont seul le principal contient l'activité. Les tubes correspondant à ce pic sont rassemblés, le volume est mesuré (ainsi que le volume mort), et l'activité est dosée. C'est également sur cette solution qu'est dosé le nombre de groupements SPDP introduits par molécule d'enzyme ainsi que la quantité de protéines (voir ci-dessous).

**E. Couplage enzyme-peptides**

On se trouve en présence d'un peptide activé à son extrémité amino-terminale par un groupement vinylique susceptible de prendre part à une copolymérisation avec les constituants d'un gel de polyacrylamide et terminé à son extrémité carboxy-terminale par un point disulfure.

On réduit tout d'abord les ponts disulfures des groupements SPDP introduits sur l'enzyme. Dans ce but, la solution d'enzyme est incubée avec 1 ml d'une solution de 15,4 mg/ml de dithiothréitol, pendant 30 min à 4°C. La solution est ensuite purifiée par filtration sur tamis moléculaire Sephadex G25 (colonne de 5,5 x 4 cm) sous le même tampon d'élution : 0,1 M NaCl 0,1 M Tris, pH 6,5. Le pic d'activité est récupéré et son pH ajusté à 0,7.

On incube alors 3,5 mg de peptides activés et lyophilisés pour 10 ml de la préparation d'ADH activé et réduit, pendant 1 à 4 h à 4°C suivant les peptides, avant de séparer les produits de la réaction par chromatographie sur tamis moléculaire dans les mêmes conditions que ci-dessus. Le pic d'activité résultant est concentré à 5 ml sur membrane Amicon PM 10, sous une pression de 1,5 bar d'azote.

**F. Copolymérisation de l'enzyme dans le gel de polyacrylamide**

Les conditions opératoires ont été définies comme suit. Nous utilisons une concentration finale en acrylamide de 12,5 % et une concentration finale relative en méthylène N,N'-bisacrylamide de 5 % par rapport à l'acrylamide; 20 µl de TEMED à 50 % et 20 µl d'une solution de persulfate d'ammonium à 12 %. Toutes les solutions sont réalisées dans de l'eau bidistillée dégazée. On y ajoute également 0,1 ml d'une solution de $NAD^+$ pour protéger le site actif de l'enzyme au cours de la polymérisation. Après 40 sec, 0,2 ml de la solution d'ADH sont ajoutés de manière à obtenir un volume final de 1,5 ml dans de petits tubes bouchés en polystyrène.

Le contenu des tubes est délicatement mélangé et ceux-ci sont plongés dans un bain de glace pendant la polymérisation (2 h).

**EXAMPLE D'APPLICATION : SYNTHESE D'ALANINE**

Nous avons voulu montrer l'application possible de l'invention dans le cadre d'un bioréacteur enzymatique à régénération de cofacteur. Nous avons choisi le système suivant :

ADH : alcool déshydrogénase

AlaDH : alanine déshydrogénase

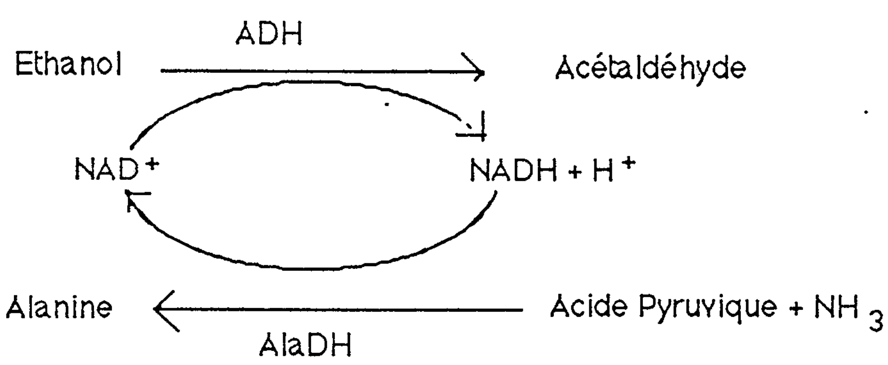


Un mg de ces enzymes activées à la polysérine a été immobilisé par copolymérisation dans un gel de polyacrylamide tel qu'expliqué dans l'invention. Ces deux enzymes ont été immobilisés dans le même gel ou

dans des gels différents.

a) Réaction dans le même gel. La solution de départ comprenait un tampon TRIS-HCl 50 mM pH 8, 5 mM EDTA, 0,47 M éthanol, 6 mM pyruvate 90,2 $MNH_4Cl$ et 3 mM $NAD^+$. La solution est recyclée continuellement sur la colonne et le dosage de l'alanine formée est réalisé par réaction à la ninhydrine.

b) Réaction dans deux gels : la solution de départ est la même, mais elle est d'abord recyclée sur la colonne contenant l'ADH avant d'être passée sur la colonne d'AlaDH où se fera la synthèse d'alanine. Le schéma automatisé de cette expérience est montré à la figure 2. Les résultats de ces 2 expériences indiquent que pour les 2 enzymes immobilisés dans une colonne, on obtenait 42 % de production d'alanine après 160 min et 47 % après 200 min dans le cas des 2 colonnes. Il faut dire qu'excepté la fixation de l'ADH par l'intermédiaire des peptides dans le gel, aucun autre paramètre n'a été optimalisé. Le montage de la figure 2 permet non seulement l'automatisation du procédé mais permettra à plus long terme en utilisant des dérivés de $NAD^+$ de gros poids moléculaire (NAD-dextran ou NAD-polyéthylènegyclol) et en incorporant entre chaque colonne une étape d'ultrafiltration, de pouvoir obtenir les produits désirés d'une enzyme sans contamination par les substrats ou produits de l'autre. Par exemple, dans le cas décrit ici, l'acétaldéhyde pourra être séparé de NADH-Dextran après la 1ère colonne et l'alanine du $NAD^+$ dextran après la 2ème colonne. Le NAD-dextran recirculant continuellement entre les deux sous-formes oxydée ou réduite. De même d'autres enzymes pourraient être utilisées pour regénérer le NADH comme l'acétaldéhyde déshydrogénase ou le système : méthanol-formaldéhyde- déshydrogénase.

## Revendications

1.- Procédé d'immobilisation d'enzymes caractérisé en ce que le micro-environnement de l'enzyme est modifié.

2.- Procédé d'immobilisation d'enzymes caractérisé en ce que le micro-environnement de l'enzyme est modifié par la présence de bras peptidiques, ce qui peut entraîner des modifications des propriétés de l'enzyme.

3.- Procédé d'immobilisation d'enzymes caractérisé en ce que le micro-environnement est modifié par la présence de molécules chimiques pouvant être un acide aminé ou un peptide, contenant un ou plusieurs groupements SH.

4.- Procédé de modification du micro-environment de l'enzyme par greffage sur celui-ci d'une ou de plusieurs chaînes peptidiques à l'exception d'acide aminé acylé sur les protéases, ce qui peut entraîner des modifications des propriétés de l'enzyme.

5.- Procédé de modification du micro-environment de l'enzyme par greffage sur celui-ci d'une ou de plusieurs chaînes peptidiques à l'exception d'acide aminé acylé sur les protéases, ce qui permet d'étudier l'influence de ce micro-environnement sur les propriétés des enzymes.

6.- Procédé de modification du micro-environment de l'enzyme par greffage sur celui-ci de molécules chimiques, pouvant être un acide aminé ou un peptide , contenant un ou plusieurs groupements SH à l'exception d'acide aminé acylé sur les protéases.

7.- Utilisation d'enzymes en solution ou immobilisés dont le micro-environnement est modifié suivant les revendications 1, 2, 3, 4, 5, 6.

8.- Utilisation d'enzymes en solution ou immobilisés dont le micro-environnement est modifié suivant les revendications 1, 2, 3, 4, 5 et 6 afin de regénérer un cofacteur.

9.- Utilisation d'enzymes en solution ou immobilisés dont le micro-environnement est modifié suivant les revendications 1, 2, 3, 4, 5, et 6 afin de réaliser des synthèses utilisant un cofacteur.

10.- Utilisation de 2 ou plusieurs enzymes en solution ou immobilisés dont le micro-environnement est modifié suivant les revendications 1, 2, 3, 4, 5 et 6 afin d'effectuer des synthèses tout en regénérant le cofacteur utilisé au cours de cette synthèse.

Figure 1.

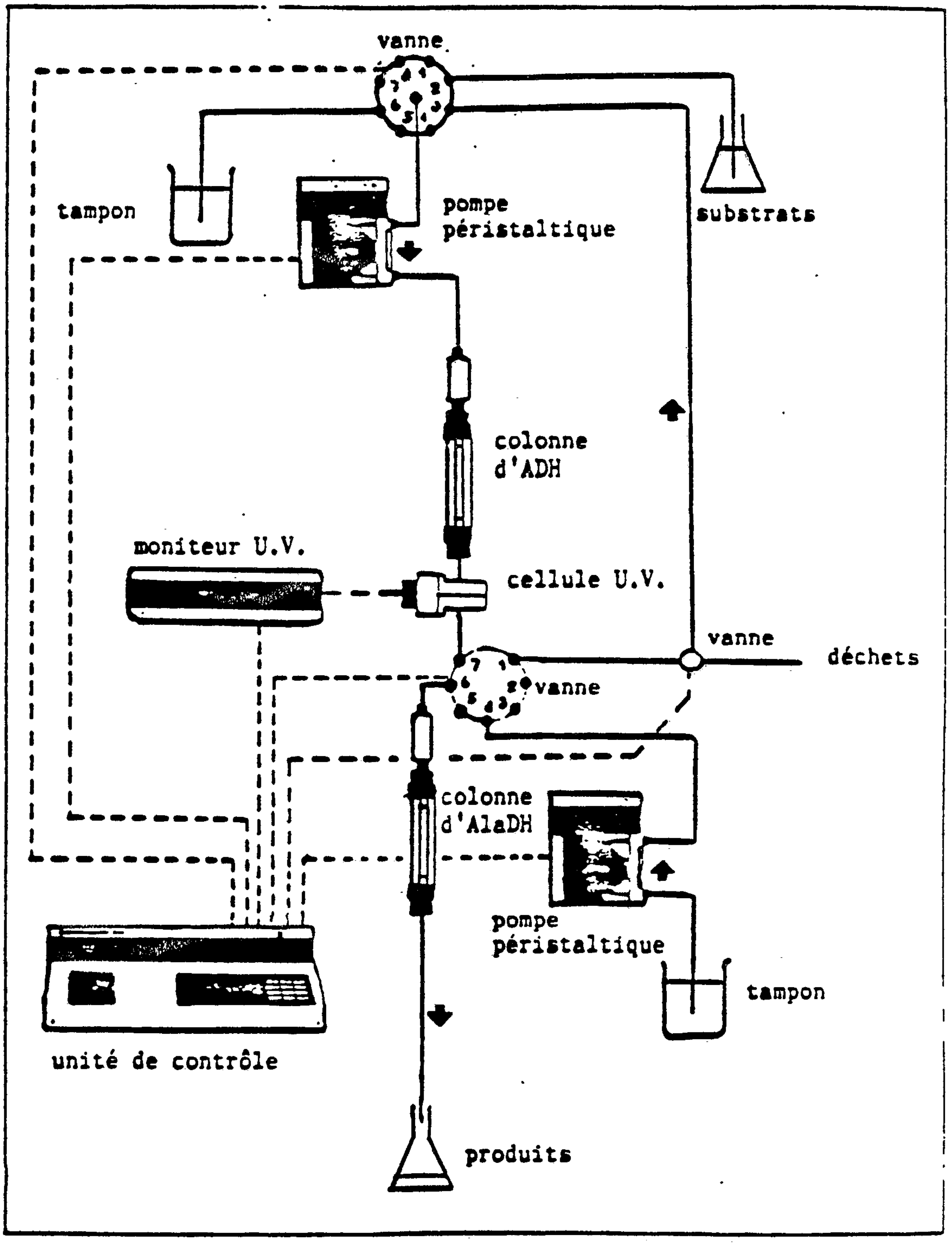
vanne
tampon
pompe
péristaltique
substrats
colonne
d'ADH
moniteur U.V.
cellule U.V.
vanne
déchets
vanne
colonne
d'AlaDH
pompe
péristaltique
tampon
unité de contrôle
produits

Office européen des brevets

# RAPPORT DE RECHERCHE EUROPEENNE

Numero de la demande

EP 88 87 0172

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-3 886 042 (BLUMBERG)<br>* Colonne 2, lignes 14,44 *<br>--- | 1-10 | C 12 N 9/96<br>C 12 N 11/06 //<br>C 12 N 9/04 |
| Y | CHEMICAL ABSTRACTS, vol. 108, no. 1, 4 janvier 1988, page 265, résumé no. 2662q, Columbus, Ohio, US; V. BILLE et al.: "Affinity and stability modifications of immobilized alcohol dehydrogenase through multipoint copolymerization", & BIOCHIM. BIOPHYS. ACTA 1987, 915(3), 393-8<br>* Résumé *<br>----- | 1-10 | |
| | | | **DOMAINES TECHNIQUES RECHERCHES (Int. Cl.4)** |
| | | | C 12 N |

Le présent rapport a été établi pour toutes les revendications

| Lieu de la recherche | Date d'achèvement de la recherche | Examinateur |
|---|---|---|
| LA HAYE | 21-02-1989 | TURMO Y BLANCO C.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)